# EUROPEAN PATENT SPECIFICATION

(11) **EP 2 964 149 B1**
(45) Date of publication and mention of the grant of the patent: **08.06.2022**
(21) Application number: 14712930.8
(22) Date of filing: 05.03.2014
(51) Int. Cl.: A61F 2/08, A61B 17/04

(54) **COMPOSITE INTERFERENCE SCREWS AND DRIVERS**
VERBUNDINTERFERENZSCHRAUBEN UND EINTREIBER
VIS ET TOURNEVIS COMPOSITES D'INTERFÉRENCE

(30) Priority: 06.03.2013 US 201313787139
(43) Date of publication of application: 13.01.2016
(73) Proprietor: Smith & Nephew, Inc., Andover, MA 01810 (US)
(72) Inventor: ARAI, Tatsuya, Houston, Texas 77058 (US); HOUSMAN, Mark Edwin, North Attleborough, Massachusetts 02760 (US); KOSKI, Matthew Edwin, Westford, Massachusetts 01886 (US)
(74) Representative: Smith & Nephew
(86) International application number: PCT/US2014/020747
(87) International publication number: WO 2014/138233

(56) References cited:
- EP-A1- 1 917 926
- EP-A1- 2 596 758
- EP-A2- 2 036 501
- EP-A2- 2 036 501
- US-A1- 2012 179 163
- US-A1- 2012 179 163

## Description

This application is a continuation-in-part application of US Patent Application Serial No. 13/044,777 filed March 10, 2011, which claims priority to US Patent Application Serial No. 61/312291 filed March 10, 2010, US Patent Application Serial No. 61/334808, filed May 14, 2010 and US Patent Application Serial No. 61/359080 June 28, 2010.

### BACKGROUND

### FIELD OF TECHNOLOGY

The present disclosure relates to medical apparatuses and procedures in general, and more particularly to medical apparatuses and procedures for reconstructing a ligament.

### RELATED ART

In many cases, ligaments are torn or ruptured as the result of an accident. Accordingly, various procedures have been developed to repair or replace such damaged ligaments.

For example, in the human knee, the anterior and posterior cruciate ligaments (i.e., the "ACL" and "PCL") extend between the top end of the tibia and the bottom end of the femur. Often, the anterior cruciate ligament (i.e., the ACL) is ruptured or torn as the result of, for example, a sports-related injury. Consequently, various surgical procedures have been developed for reconstructing the ACL so as to restore substantially normal function to the knee.

In many instances, the ACL may be reconstructed by replacing the ruptured ACL with a graft ligament. More particularly, in such a procedure, bone tunnels are generally formed in both the top of the tibia and the bottom of the femur, with one end of the graft ligament being positioned in the femoral tunnel and the other end of the graft ligament being positioned in the tibial tunnel, and with the intermediate portion of the graft ligament spanning the distance between the bottom of the femur and the top of the tibia. The two ends of the graft ligament are anchored in their respective bone tunnels in various ways well known in the art so that the graft ligament extends between the bottom end of the femur and the top end of the tibia in substantially the same way, and with substantially the same function, as the original ACL. This graft ligament then cooperates with the surrounding anatomical structures so as to restore substantially normal function to the knee.

In some circumstances, the graft ligament may be a ligament or tendon which is harvested from elsewhere within the patient's body, e.g., a patella tendon with or without bone blocks attached, a semitendinosus tendon and/or a gracilis tendon.

As noted above, various approaches are well known in the art for anchoring the two ends of the graft ligament in the femoral and tibial bone tunnels.

In one well-known procedure, which may be applied to femoral fixation, tibial fixation, or both, the end of the graft ligament is placed in the bone tunnel, and then the graft ligament is fixed in place using a headless orthopedic screw, generally known in the art as an "interference" screw. More particularly, with this approach, the end of the graft ligament is placed in the bone tunnel and then the interference screw is advanced into the bone tunnel so that the interference screw extends parallel to the bone tunnel and simultaneously engages both the graft ligament and the side wall of the bone tunnel. In this arrangement, the interference screw essentially drives the graft ligament laterally, into engagement with the opposing side wall of the bone tunnel, whereby to secure the graft ligament to the host bone with a so-called "interference fit". Thereafter, over time (e.g., several months), the graft ligament and the host bone grow together at their points of contact so as to provide a strong, natural joinder between the ligament and the bone.

Interference screws have proven to be an effective means for securing a graft ligament in a bone tunnel. However, the interference screw itself generally takes up a substantial amount of space within the bone tunnel, which can limit the surface area contact established between the graft ligament and the side wall of the bone tunnel. This in turn limits the region of bone-to-ligament in-growth, and hence can affect the strength of the joinder. By way of example but not limitation, it has been estimated that the typical interference screw obstructs about 50% of the potential bone-to-ligament integration region.

For this reason, substantial efforts have been made to provide interference screws fabricated from absorbable materials, so that the interference screw can eventually disappear over time and bone-to-ligament in-growth can take place about the entire perimeter of the bone tunnel. To this end, various absorbable interference screws have been developed which are made from biocompatible, bioabsorbable polymers, e.g., polylactic acid (PLA), polyglycolic acid (PGA), etc. These polymers generally provide the substantial mechanical strength needed to advance the interference screw into position, and to thereafter hold the graft ligament in position while bone-to-ligament in-growth occurs, without remaining in position on a permanent basis.

In general, interference screws made from such biocompatible, bioabsorbable polymers have proven clinically successful. However, these absorbable interference screws still suffer from several disadvantages. First, clinical evidence suggests that the quality of the bone-to-ligament in-growth is somewhat different than natural bone-to-ligament in-growth, in the sense that the aforementioned bioabsorbable polymers tend to be replaced by a fibrous mass rather than a well-ordered tissue matrix. Second, clinical evidence suggests that absorption generally takes a substantial period of time, e.g., on the order of three years or so. Thus, during this absorption time, the bone-to-ligament in-growth is still significantly limited by the presence of the interference screw. Third, clinical evidence suggests that, for many patients, absorption is never complete, leaving a substantial foreign mass remaining within the body. This problem is exacerbated somewhat by the fact that absorbable interference screws generally tend to be fairly large in order to provide them with adequate strength, e.g., it is common for an interference screw to have a diameter (i.e., an outer diameter) of 8-12 mm and a length of 20-25 mm. US20120179163 discloses a delivery device and screw combination.

Thus, there is a need for a new and improved interference fixation system which (i) has the strength needed to hold the graft ligament in position while bone-to-ligament in-growth occurs, and (ii) promotes superior bone-to-ligament in-growth.

### SUMMARY

The invention is defined in appended claim 1. In one aspect, the present disclosure relates to an anchor. The anchor includes a suture bridge having a proximal end and distal end. The distal end of the suture bridge has a thickness greater than a thickness of the proximal end of the suture bridge. At least two ribs extend from the proximal end of the suture bridge to a proximal end of the anchor. At least one open helical coil wraps around the at least two ribs and extends, substantially, from the proximal end of the suture bridge to the proximal end of the anchor. The at least one open helical coil defines an internal volume communicating with a region exterior to the anchor through apertures between turns of the at least one open helical coil. The at least two ribs are engagable with a grooved shaft of a driver.

In yet another aspect, the present disclosure relates to a delivery device and anchor combination. The delivery device of the combination includes a handle and shaft connected to the handle. The shaft includes a distal end having a slot and at least two grooves extending from the slot. The anchor of the combination includes a suture bridge having a proximal end and distal end. The distal end of the suture bridge has a thickness greater than a thickness of the proximal end of the suture bridge. At least two ribs extend from the proximal end of the suture bridge to a proximal end of the anchor. At least one open helical coil wraps around the at least two ribs and extends, substantially, from the proximal end of the suture bridge to the proximal end of the anchor. The at least one open helical coil defines an internal volume communicating with a region exterior to the anchor through apertures between turns of the at least one open helical coil. The anchor is located on the distal end of the delivery device such that the slot houses the proximal portion of the suture bridge and the at least two grooves engage the at least two ribs of the suture bridge.

Further areas of applicability of the present disclosure will become apparent from the detailed description provided hereinafter. It should be understood that the detailed description and specific examples, while indicating the preferred embodiment of the disclosure, are intended for purposes of illustration only and are not intended to limit the scope of the disclosure.

### BRIEF DESCRIPTION OF THE DRAWINGS

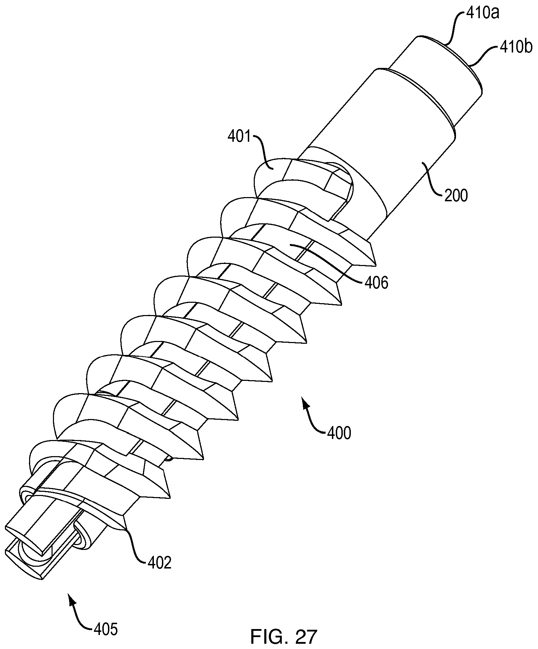

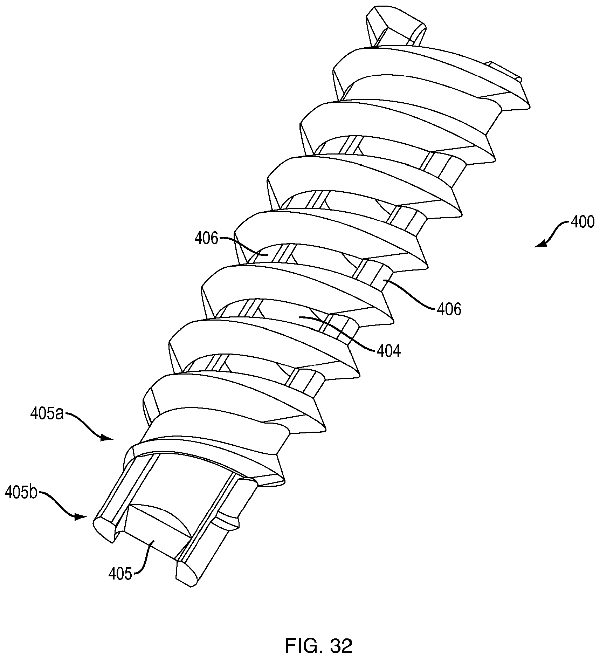

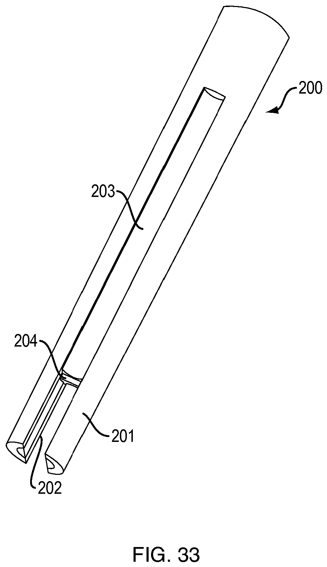

The accompanying drawings, which are incorporated in and form a part of the specification, illustrate examples of the present disclosure and together with the written description serve to explain the principles, characteristics, and features of the disclosure. Embodiments of the invention are disclosed in Figs. 27 to 33. The other figures disclose examples that do not fall under the scope of the claims. In the drawings:
Fig. 1 shows a first example delivery device.
Fig. 2 shows a side view of the shaft of the delivery device of Fig. 1.
Fig. 2A shows an exploded view of the distal end of the shaft of Fig. 2.
Fig. 3 shows a cross-sectional view of the shaft of Fig. 2.
Fig. 4 shows a front view of the distal end of the shaft of Fig. 2.
Fig. 5 shows an isometric view of the screw for use with the shaft of Fig. 2.
Fig. 6 shows a side view of the screw of Fig. 5.
Fig. 7 shows a cross-sectional view of the screw of Fig. 6.
Fig. 8 shows a second example of a shaft.
Fig. 9 shows a side view of the inner member of the shaft of Fig. 8.
Fig. 9A shows an exploded view of the distal end of the inner member of Fig. 9.
Fig. 10 shows a cross-sectional view of the inner member of the shaft of Fig. 9.
Fig. 11 shows a front view of the distal end of the inner member of Fig. 9.
Fig. 12 shows an isometric view of the outer member of the shaft of Fig. 8.
Fig. 13 shows a cross-sectional view of the outer member of Fig. 12.
Figs. 14 and 15 show side views of the shaft of Fig. 8 with the outer member in different positions.
Fig. 16 shows an isometric view of a third example of a shaft and a screw for use with the shaft.
Fig. 17 shows an isometric view of the shaft of Fig. 16.
Fig. 18 shows an isometric view of the screw of Fig. 16.
Fig. 19 shows a side view of the screw of Fig. 16.
Fig. 20 shows a cross-sectional view of the screw of Fig. 19.
Fig. 21 shows an isometric view of a fourth example of a shaft and a screw for use with the shaft.
Fig. 22 shows an isometric view of the screw of Fig. 21.
Fig. 23 shows an isometric view of the shaft of Fig. 21.
Fig. 24 shows an isometric view of the shaft of Fig. 21 and an alternative screw for use with the shaft.
Fig. 25 shows a side view of the screw of Fig. 24.
Fig. 26 shows a cross-sectional view of the screw of Fig. 24.
Fig. 27 shows an isometric view of an example anchor with suture bridge and example inserter shaft for the anchor in accordance with the invention.
Fig. 28 shows a side view of the shaft and anchor of Fig. 27.
Fig. 29 shows a cross-sectional view of Fig. 28.
Fig. 30 shows a close up view of the distal end of the anchor of Fig. 29.
Fig. 31 shows a cross-sectional view of the anchor of Fig. 27.
Fig. 32 shows an isometric view of the anchor of Fig. 27.
Fig. 33 shows an isometric view of the shaft of Fig. 27.
Fig. 34 shows a cross-sectional view of an anchor inserted into bone.
Fig. 35 shows an isometric view of an example anchor with suture bridge and proximal reinforcement.
Fig. 36 shows an isometric view of another example anchor with suture bridge.
Fig. 37 shows an isometric view of yet another example anchor with suture bridge.
Fig. 38 shows a cross-sectional view of the anchor of Fig. 37.

### DETAILED DESCRIPTION OF THE EMBODIMENTS

The following description of the examples is merely exemplary in nature and is in no way intended to limit the disclosure, its application, or uses.

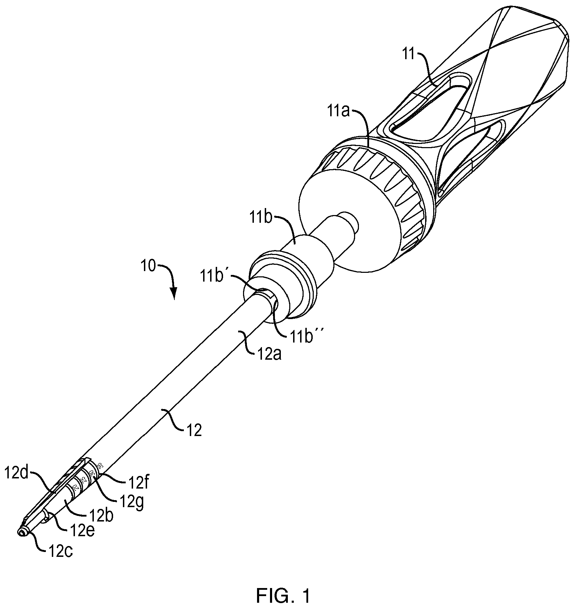

Fig. 1 shows a first example delivery device **10**. The device **10** includes a handle assembly **11** and a shaft **12** coupled to the handle assembly **11.** The handle assembly **11** includes a handle **11a** and a connector **11b** coupled to the handle **11a.** The connector **11b** has a channel **11b'** and an opening **11b"** to the channel **11b'.** The opening **11b"** is in the shape of a "D". A proximal end **12a** of the shaft **12** is disposed within the channel **11b'.**

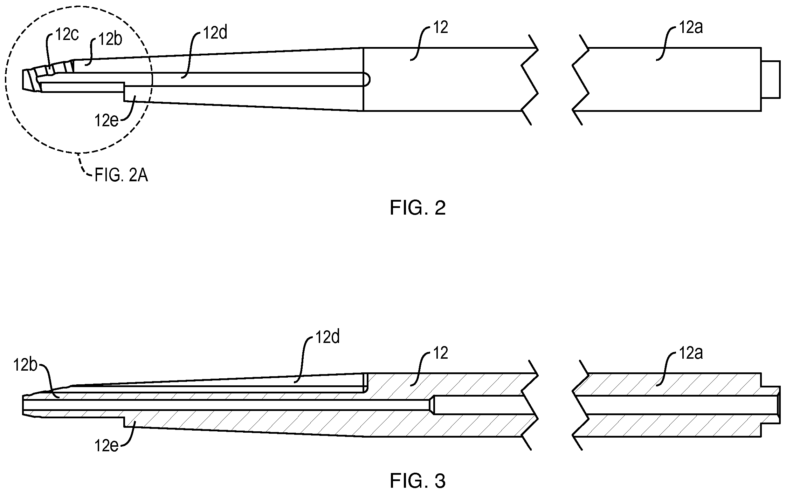

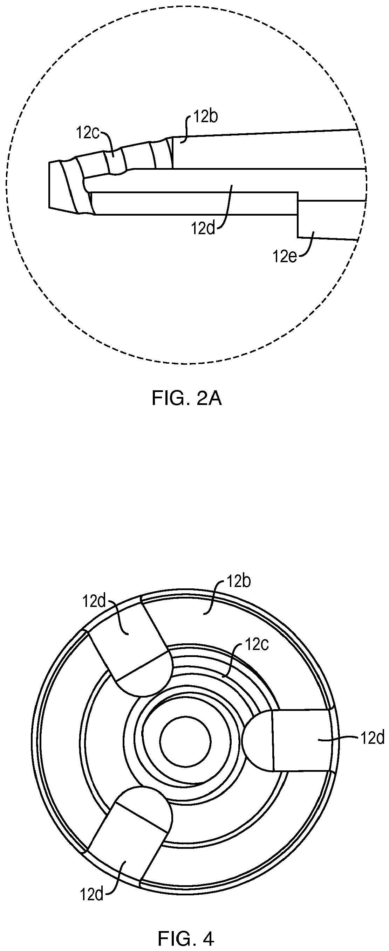

Figs. 2, 2A, and 3-4 show the shaft **12**. The shaft **12** includes a proximal end **12a** and a distal end **12b**. The proximal end **12a** is in the shape of a "D" to match the shape of the opening **11b".** The distal end **12b** includes threads **12c**, grooves **12d**, and a depth stop **12e**. The grooves **12d** extend a partial length of the shaft **12** and intersect the threads **12c**. The depth stop **12e** is for use with a depth stop on a screw that the device **10** is used to implant into a bone tunnel during ligament reconstruction surgery.

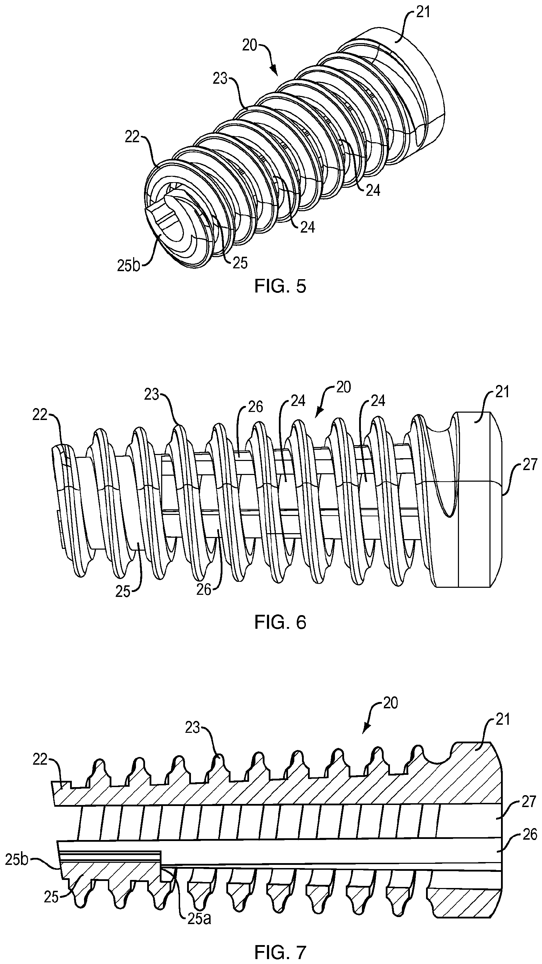

Figs. 5-7 show the screw **20** for use with the delivery device **10** of the present disclosure. The screw **20** includes a proximal end **21** and a distal end **22**. A majority of the screw **20** includes screw threads **23** in the form of an open helical coil, i.e. a connected series of continuous regularly spaced turns extending in a helical or spiral form substantially from the proximal end **21** to the distal end **22** with apertures **24** being defined by the space between the turns of the coil. In other words, interference screw **20** may include an open helical coil defining an internal volume, with the internal volume communicating with the region exterior to the open helical coil through the spacing between the turns of the open helical coil. The distal end **22** also includes a depth stop **25** that extends a partial length of the screw **20.** The depth stop **25** includes a proximal end **25a** and a distal end **25b.** Additionally, a plurality of longitudinally-extending runners **26** extend along the interior of the screw threads **23.**

The distal end **12b** of the shaft **12** is placed within the interior of the screw **20,** via the opening **27,** until the proximal end **25a** of the depth stop **25** engages the depth stop **12e** of the shaft **12.** During insertion of the shaft **12** into the screw **20,** the runners **26** engage the grooves **12d** and become housed within the grooves **12d.** As shown in Fig. 1, the distal end **12b** of the shaft **12** also includes hash marks **12f,** each of which is associated with a number **12g.** Once the screw **20** is placed on the shaft **12,** the proximal end **21** of the screw **20** aligns with one of the hash marks/numbers **12f,** thereby indicating the length of the screw **20.**

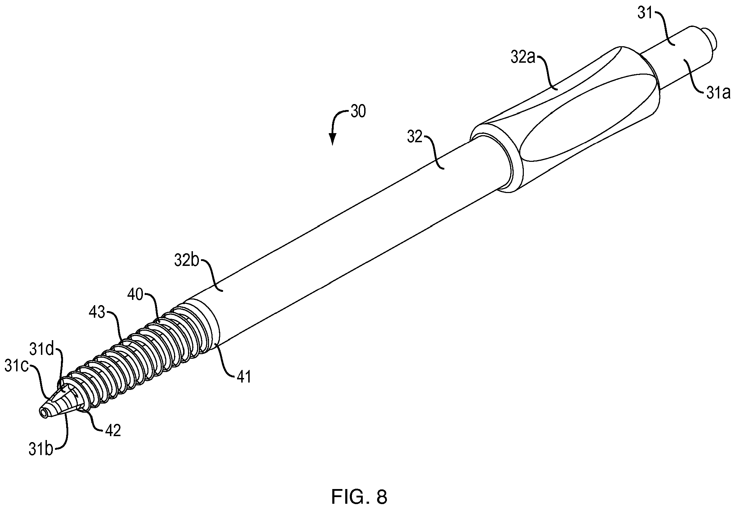

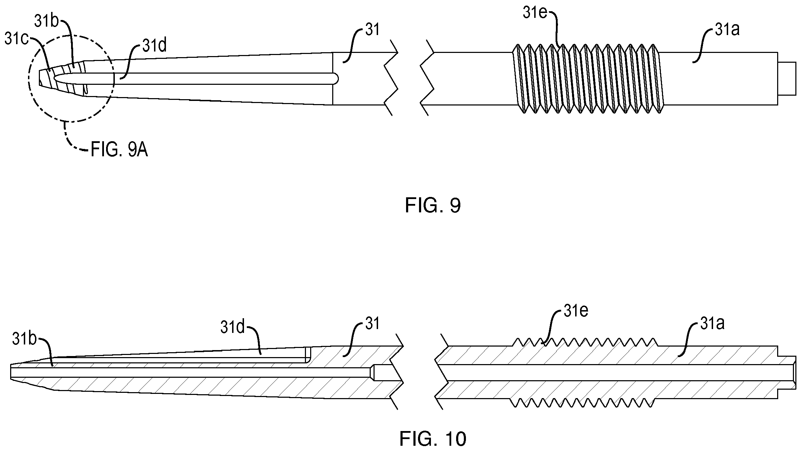

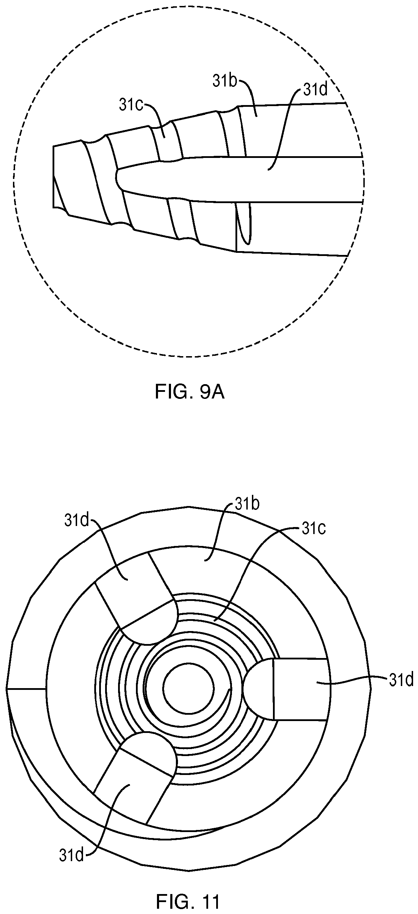

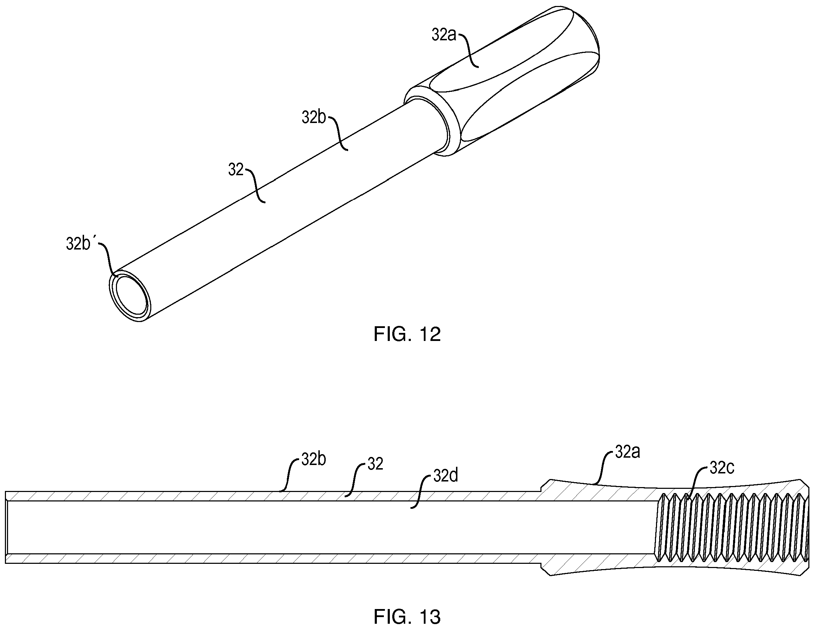

Figs. 8, 9-9A, and 10-15 show an alternative shaft **30** of the present disclosure. The shaft **30** includes an inner member **31** and an outer member **32** disposed over the inner member **31.** The proximal end **31a** of the inner member **31** is similar in shape to the proximal end **12a** of the shaft **12.** The distal end **31b** of the inner member **31** includes threads **31c.** Grooves **31d** extend along the member **31** and intersect the threads **31c.** Additionally, threads **31e** are located between the proximal and distal ends **31a,31b** of the member **31.** The outer member **32** includes a first section **32a** and a second section **32b.** The first section **32a** has a larger diameter than the second section **32b.** The first section **32a** also includes threads **32c** on an inner wall **32d** of the outer member **32.**

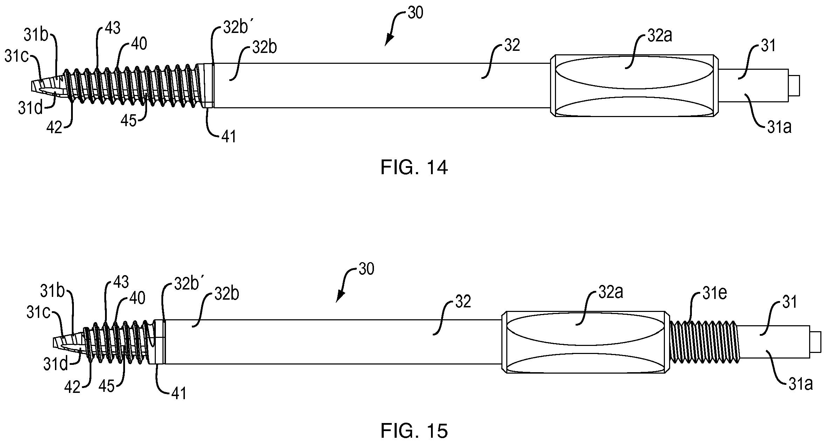

Once the outer member **32** is disposed over the inner member **31,** threads **32c** engage threads **31e** to move the outer member **32** relative to the inner member **31.** Moving the outer member **32** relative to the inner member **31** allows for more or less of the distal end **31b** of the inner member **31** to be shown. Similar to the distal end **12b** of the shaft **12,** the distal end **31b** of inner member **31** includes hash marks/numbers **(not shown)** that align with an end **32b'** of the second section **32b,** thereby indicating a length of screw **40** that will be disposed on the distal end **31b** of the inner member **31.** As shown in Figs. 14 and 15, the outer member **32** is located at different positions along the length of the inner member **31** to allow for screws **40** of different lengths to be loaded on the distal end **31b** of the inner member **31.**

A handle assembly, similar to the handle assembly **11,** is coupled to the proximal end **31a** of the inner member **31.** Similar to screw **20,** screw **40** includes a proximal end **41** and a distal end **42.** The screw **40** includes screw threads **43** in the form of an open helical coil having an interior and a plurality of longitudinally-extending runners **45** extending along the interior of the screw threads **43.** Screw **40** is more fully described in United States Patent Application Publication No. 20080154314. Once the outer member **32** has been moved to indicate the screw length, the screw **40** is loaded onto the distal end **31b,** such that a proximal end **41** of the screw **40** engages the end **32b'** and the runners **45** engage the grooves **31d** and become housed within the grooves **31d.**

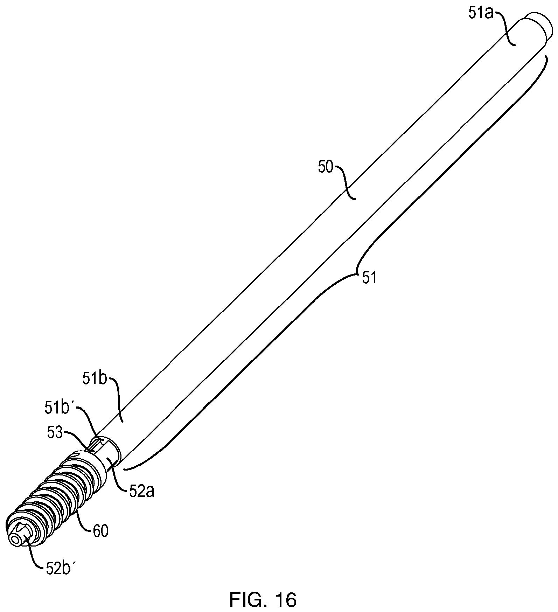

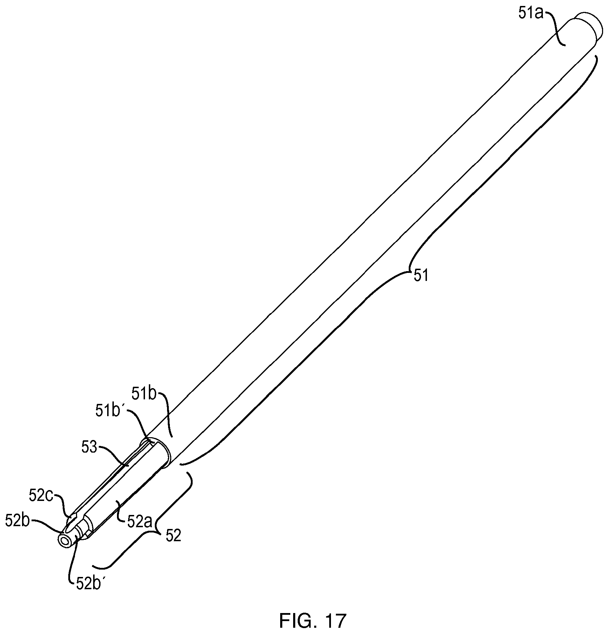

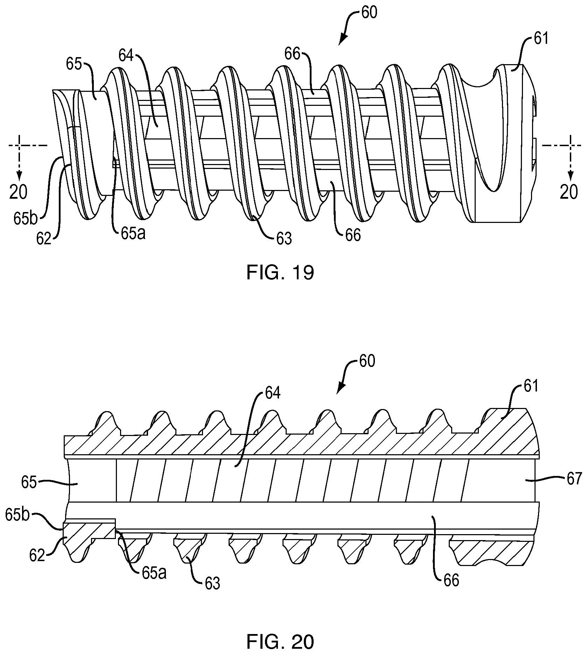

Figs. 16-20 show another example shaft **50** and screw **60.** The shaft **50** includes a first portion **51** including a proximal end **51a** and a distal end **51b** and a second portion **52** including a first area **52a** and a second area **52b.** The proximal end **51a** is configured to be coupled to a handle assembly, similar to the handle assembly **11.** However, other handle assemblies may be used. The first area **52a** has a smaller diameter than the first portion **51,** such that a first depth stop **51b'** exists at the distal end **51b** of the first portion **51.** The second area **52b** has a smaller diameter than the first area **52a** such that a second depth stop **52c** exists between the first area **52a** and the second area **52b.** An end **52b'** of the second area **52b** is tapered to allow for easier insertion of the anchor **60** into a bone during ligament reconstruction surgery, as will be further described below. The second portion **52** also includes grooves **53** extending between the first and second areas **52a,52b**. For the purposes of this disclosure, there are three grooves **53.** However, the second portion **52** may include a higher or lower number of grooves **53.**

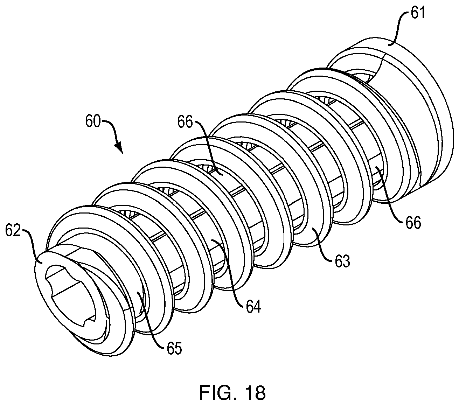

Similar to screw **20** shown in Figs. 5-7, screw **60** includes a proximal end **61** and a distal end **62.** A majority of the screw **60** includes screw threads **63** in the form of an open helical coil, i.e. a connected series of continuous regularly spaced turns extending in a helical or spiral form substantially from the proximal end **61** to the distal end **62** with apertures **64** being defined by the space between the turns of the coil. In other words, interference screw **60** may include an open helical coil defining an internal volume, with the internal volume communicating with the region exterior to the open helical coil through the spacing between the turns of the open helical coil. The distal end **62** also includes a depth stop **65** that extends a partial length of the screw **60.** The depth stop **65** includes a proximal end **65a** and a distal end **65b.** Unlike the open depth stop **25** of screw **20** most clearly shown in Fig. 5, the depth stop **65** of screw **60** is a closed depth stop, most clearly shown in Fig. 18. Additionally, a plurality of longitudinally-extending runners **66** extend along the interior of the screw threads **63.**

The second portion **52** of the shaft **50** is placed within the interior of the screw **60,** via the opening **67,** until the proximal end **65a** of the depth stop **65** engages the second depth stop **52c** of the shaft **50.** During insertion of the shaft **50** into the screw **60,** the runners 66 engage the grooves **53** and become housed within the grooves **53.** The screws **60** may be of a variety of lengths. For example, a screw **60** may be of such length that its proximal end **61** would engage the first depth stop **51b'**.

As described above, during ligament reconstruction surgery, the end of the graft ligament is placed in the bone tunnel and then the interference screw **20,40,60** is advanced into the bone tunnel via the use of shafts **12,30,50** so that the interference screw **20,40,60** extends parallel to the bone tunnel and simultaneously engages both the graft ligament and the side wall of the bone tunnel. The screws **20,40,60** may be used in either the femoral or tibial tunnels. Methods of ligament reconstruction via use of the screws **20,40,60** is further shown in the '314 publication shown above.

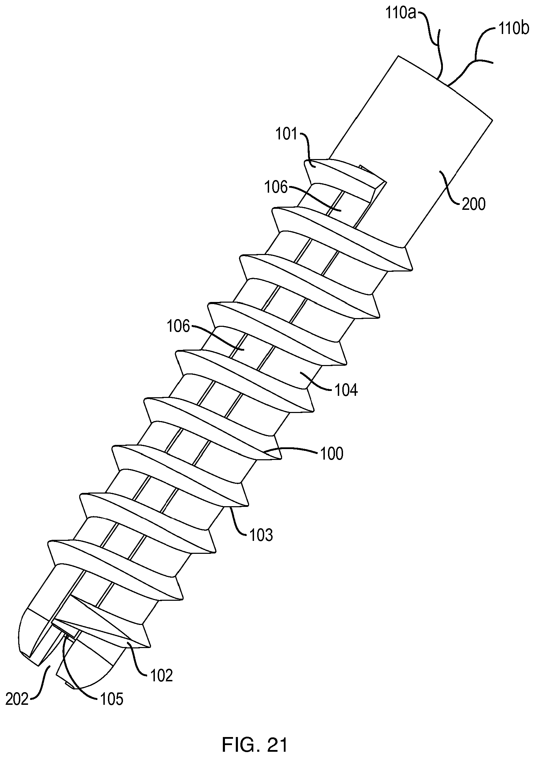

Figs. 21-23 show yet another example screw **100** and delivery device **200.** The screw **100** includes a proximal end **101** and a distal end **102.** A majority of the screw **100** includes screw threads **103** in the form of an open helical coil, i.e. a connected series of continuous regularly spaced turns extending in a helical or spiral form substantially from the proximal end **101** to the distal end **102** with apertures **104** being defined by the space between the turns of the coil. In other words, interference screw **100** may include an open helical coil defining an internal volume, with the internal volume communicating with the region exterior to the open helical coil through the spacing between the turns of the open helical coil. The distal end **102** also includes a suture bridge **105** that extends a partial length of the screw **100.** The suture bridge **105** includes a proximal end **105a** and a distal end **105b.** The distal end **105b** includes a concave shape. A flexible member **110,** such as a suture, is housed within the screw **100,** such that the suture **110** extends around the distal end **105b** of the bridge **105.** Additionally, longitudinally-extending runners **106** extend from the suture bridge **105** and along the interior of the screw threads **103.** For the purposes of this disclosure, there are two longitudinally extending runners **106.** However, more or less than two runners are within the scope of this disclosure.

The delivery device **200** includes a distal end **201** having a slot **202** and grooves **203** extending from the slot **202** on each side of the device **200.** As shown in Fig. 21, the screw **100** is located on the distal end **201** such that the suture bridge **105** is housed within the slot **202** and the runners **106** are housed within the grooves **203.** The delivery device **200** is cannulated, such that when the screw **100** is located on the device **200,** the suture ends **110a,110b** extend through the cannulation **204.**

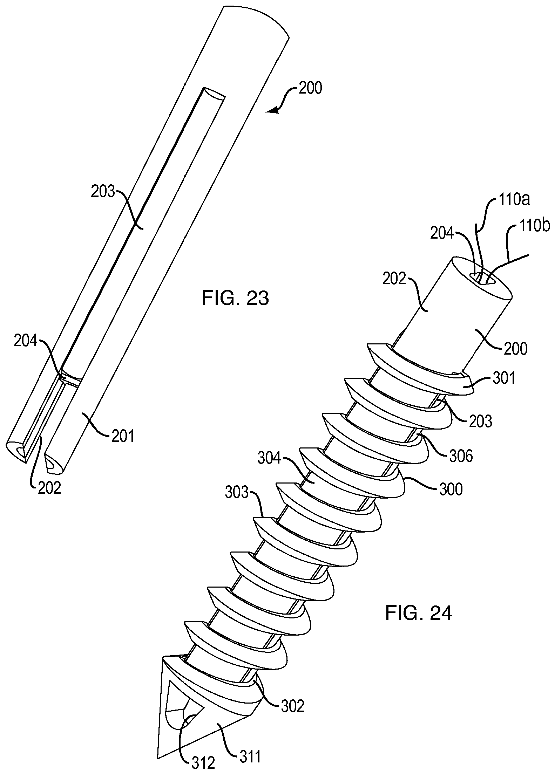

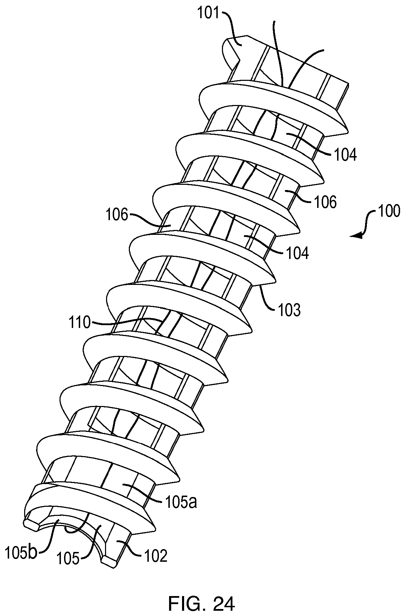

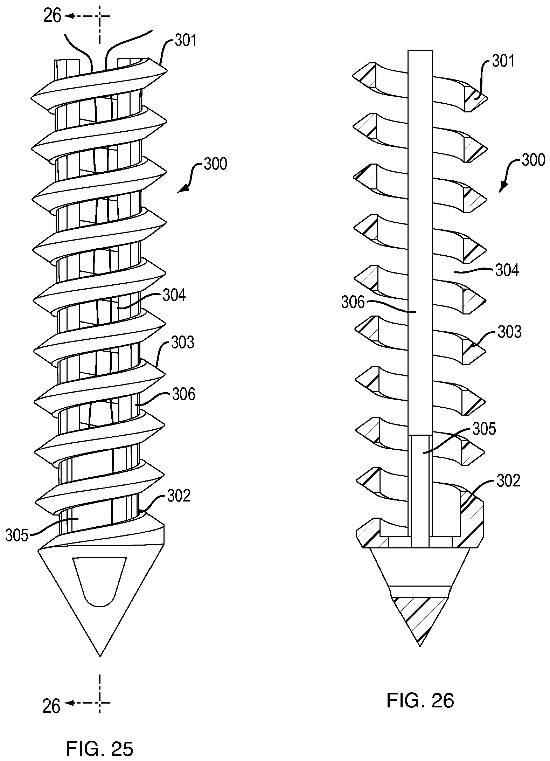

Figs. 24-26 show a screw **300** similar to screw **100.** However, screw **300** additionally includes a pointed tip **311** located on the distal end **302.** The tip **311** includes a through hole **312.** The hole **312** helps in locating the suture **110** within the interior of the screw **300.** As shown in Fig. 24, the screw **300** is located on the distal end **201** of delivery device **200** such that the suture bridge **305** is housed within the slot **202** and the runners **306** are housed within the grooves **203.** As stated above, the delivery device **200** is cannulated, such that when the screw **300** is located on the device **200,** the suture ends **110a,110b** extend through the cannulation **204,** as shown in Fig. 24.

For clarity purposes, only the distal end **201** of the device **200** is shown. However, the device **200** would include a proximal end, similar to the devices above, which may be coupled to a handle assembly, similar to handle assembly **11** above. The screws **100,300** are used in the repair of soft tissue, specifically to re-attach tissue to bone. One example of this repair is when the screw **100,300** is delivered into bone via the use of device **200,** the device **200** is removed from screw **100,300,** the tissue is placed on the bone to be adjacent the screw **100,300,** the suture ends **110a,110b** are pulled through the tissue, and then the suture ends **110a,110b** are tied. A hole may be made in the bone prior to insertion of the screw **100,300** into the bone. However, screw **300** may be inserted into bone without first making a hole in the bone. In this case, the pointed tip **311** is used to start insertion of the screw **300** into the bone and then rotary motion may be used to complete insertion of the screw **300** into the bone. Other methods of tissue repair via use of these screws and delivery device may also be used.

The handle **11a** of handle assembly **11** is made from plastic, however, other non-metal and metal materials may also be used. The shape and size of handle **11a** may be any shape and size necessary to help facilitate insertion of the screw **20** into bone. The coupler **11b** is made from a metal material, such as stainless steel or titanium, but may be made from other metal and non-metal materials that are strong enough to withstand the forces applied during surgery. The coupler **11b** is press-fit to the handle **11a,** but may be coupled to the handle **11a** in any other manner known to those of skill in the art. The size and shape of the coupler **11b** may be any size and shape necessary to help facilitate insertion of the screw **20** into bone. The channel **11b'** may be any length necessary and the opening **11b"** may be any shape necessary to facilitate coupling of the shaft **12** to the coupler **11b.**

The shaft **12** is made from a metal material, such as stainless steel and titanium,
however, other metal and non-metal materials that would withstand the forces applied during surgery may be used. The diameter of the shaft **12** may vary. The proximal end **12a** of the shaft **12** may be any shape necessary to facilitate insertion of the end **12a** through opening **11b"** and into channel **11b'.** The number of threads **12c** and grooves **12d** may vary and the lengths of the grooves **12d** may also vary. The location of depth stop **12e** may also vary based on the diameter of the shaft **12** and the diameter of the screw **20** that is used. The grooves **12d,** depth stop **12e,** and threads **12c** may be formed by any method known to one of skill in the art.

The screw **20** is made from a polymer material via a molding method. However, other material, which would allow the screw **20** to withstand forces applied during surgery, and other methods of making may be used. The depth stop **25** is open ended and doesn't extend the entire inner diameter of the screw **20.** The amount of screw inner diameter that the depth stop **25** covers may vary and the length of the depth stop **25** may vary based on the diameter of the screw. The number and length of the runners **26** may also vary. Once the screw **20** is located on the shaft **12,** the distal end **12b** of the shaft **12** extends from the distal end **22** of the screw **20.** During insertion of the screw **20** into bone, the threads **12c** create threads in the bone, thereby creating a seat for the screw threads **23,** as described more fully in the '314 publication. The amount of the distal end **12b** of the shaft **12** that extends from the distal end **22** of the screw **20** may vary.

The diameters of the first and second sections **32a,32b** of outer member 32 may vary and the number of threads **32c** may also vary. The number of threads **31c,31e** and grooves **31d** may vary and the lengths of the grooves **31d** may also vary. The inner and outer members **31,32** are made from a metal material, such as stainless steel and titanium, and via a method known to one of skill in the art. However, other materials may also be used. The screw **40** is made from a polymer material via a molding method. However, other material and methods of making may be used. The number and length of the runners **45** may also vary. Once the screw **40** is located on the shaft **30,** the distal end **31b** of the shaft **30** extends from the distal end **42** of the screw **40.** During insertion of the screw **40** into bone, the threads **31c** create threads in the bone, thereby creating a seat for the screw threads **43,** as described more fully in the '314 publication. The amount of the distal end **31b** of the shaft **30** extending from the screw **40** may vary.

The shaft **50** is made from a metal material, such as stainless steel or titanium, but may be made from another metal material or a non-metal material that is strong enough to withstand the force applied to the shaft **50** during surgery. The shaft **50** may be made via a method known to one of skill in the art. The diameters of the first and second portions **51,52** may vary along with the number and lengths of the grooves **53** and the locations of the depth stops **52c,51b'** may vary based on the diameter of the screw **60** or other factors. Rather than being tapered, the end **52b'** may be designed in another manner to allow easier insertion of the screw **60** into bone. The screw **60** is made from a polymer material via a molding method. However, other material, which would allow the screw to withstand the forces applied during surgery, and other methods of making may be used. The number and length of the runners **66** may also vary. Once the screw **60** is located on the shaft **50,** the second portion **52** of the shaft **50** extends from the distal end **62** of the screw **60.** The amount of the second portion **52** extending from the screw **60** may vary. Additionally, the length of the depth stop **65** may also vary based on the diameter of the screw **60** or other factors.

The delivery device **200** is made from a metal material, such as stainless steel or titanium, but may be made from a non-metal material that is strong enough to withstand the forces applied to the device **200** during surgery. The delivery device **200** is made via a method known to one of skill in the art. The screws **100,300** are made from a polymer material and via a molding process, however, other material, which would allow the screw to withstand the forces applied during surgery, and other processes known to one of skill in the art may be used. The suture bridge **105** may have a distal end **105b** having a shape other than concave and the length of the suture bridge **105,** the slot **202,** and the grooves **203** may vary. The size and the shape of the hole **312** may vary.

For example, Figs. 27-33 show an embodiment of a screw (anchor) **400** and the delivery device **200** of the present invention. The screw **400** includes a proximal end **401** and a distal end **402.**

The distal end **402** also includes a suture bridge **405** that extends a partial length of the screw **400.** The suture bridge **405** includes a proximal end **405a** and a distal end **405b.**

The distal end **405b** of the suture bridge **405** has a thickness greater than a thickness of the proximal end **405a** of the suture bridge **405.** In one example, the distal end **405b** includes a convex shape. A convenient example of the screw **400** has a suture bridge with a bulbous profile. A flexible member **410,** such as a suture, is housed within the screw **400,** such that the suture **110** extends around the distal end **405b** of the bridge **405.**

A majority of the screw **400** includes screw threads **403** in the form of an open helical coil, i.e. a connected series of continuous regularly spaced turns extending in a helical or spiral form substantially from the proximal end **405a** of the suture bridge **405** to the proximal end **401** of the screw **400** with apertures **404** being defined by the space between the turns of the coil. In other words, the screw **400** may include an open helical coil defining an internal volume, with the internal volume communicating with the region exterior to the open helical coil through the spacing between the turns of the open helical coil.

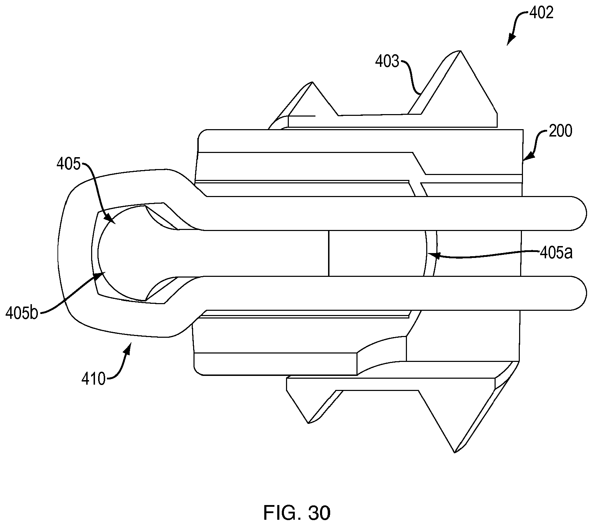

In one example of the screw **400,** the screw threads **403** cover the proximal end **405a** of the suture bridge **405** (best seen in Fig. 30). In another example of the screw **400,** the screw threads **403** start at proximal end **105a** of the suture bridge **105.** In some examples, the screw threads **403** may define, at least in part, an anchor body and may be referred to as such.

Longitudinally-extending runners (ribs) **406** extend from the suture bridge **405** and along the interior of the screw threads **403.** For the purposes of this disclosure, there are two longitudinally extending runners **406.** However, more or less than two runners are within the scope of this disclosure.

The delivery device **200** includes a distal end **201** having a slot **202** and grooves **203** extending from the slot **202** on each side of the device **200.** As shown in Fig. 27, the screw **400** is located on the distal end **201** such that the proximal end **405a** of the suture bridge **405** is housed within the slot **202** and the runners **406** are housed within the grooves **203.** The delivery device **200** is cannulated such that when the screw **400** is located on the device **200,** the suture ends **410a, 410b** extend through the cannulation **204.**

The general suture bridge design described above with reference to FIGs. 27-33 may be advantageous to screws (anchors) made from bioabsorbable material. Compared to other materials, such as polyetheretherketone (PEEK), a bioabsorbable material is weaker and more brittle. Testing shows that other suture bridge designs, while adequate for devices made from PEEK, do not provide sufficient bridge strength for products made from bioabsorbable material.

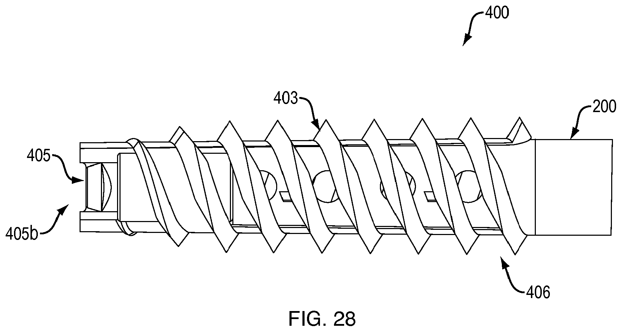

Figs. 28-31 show a convenient example of the suture bridge **405** suitable for an open-architecture anchors (e.g., fenestrated anchor) fabricated from bioabsorbable material.

Fig. 28 shows a working example of a device **400** in which a bulbous distal end **405b** of the suture bridge **405** is outside an inserter **200.** The foregoing arrangement allows additional space/volume for the suture bridge **405** to occupy and for the device **400** to still accommodate a full suture load.

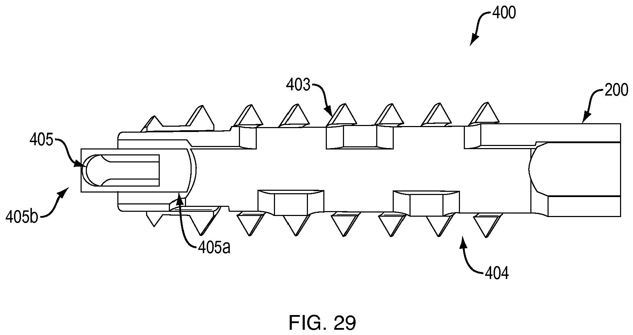

Fig. 29 shows a cross section view of working end of the device **400** - the same section of the device depicted in Fig. 28. In this case, the cross section is rotated 90 degrees. The bulbous distal portion **405b** of the suture bridge **405** protrudes outside the inserter **200** and is enlarged to better distribute the load imparted onto the suture bridge **405** by a suture.

Fig. 30 shows a close up view of the distal end **402** of the device **400.** The bulbous portion **405b** of the suture anchor **405** is outside the envelope of the inserter **200.** The extension of the bulbous portion **405b** of the bridge outside **405** of the inserter **200** allows there to be room for a suture **410** to occupy.

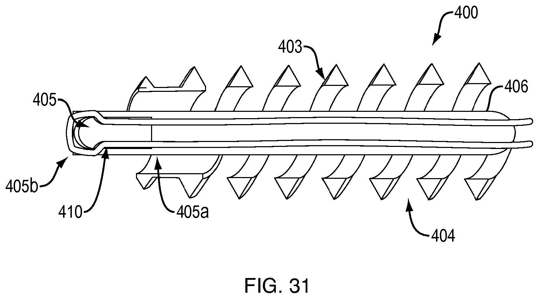

Fig. 31 shows a cross section view of the device **400** removed from the inserter **200.** The bulbous portion **405b** of the suture anchor **405** can be seen, as can the interaction between the suture **410,** suture bridge **405** and the anchor body **403.**

While the suture bridge **405** and its examples are described above in the context of a single suture, the foregoing disclosure also applies to a device loaded with multiple sutures (e.g., three) and the associated suture load. Because the distal (thick) end of the suture bridge **405** extends beyond the inserter **200,** the suture bridge **405** is able to accommodate a large suture load. Because the distal end **402** of the bridge **405** is bulbous, the suture bridge **405** can withstand significant loads applied by multiple sutures.

The general suture bridge design contemplates other variations providing a suture bridge that is structurally strong to hold up to loads imparted onto it by a suture(s), particularly during knot tying by a surgeon. In one example, the bulbous portion of the suture bridge extends distally further increasing the load carrying capability of the suture bridge. In other example, the diameter of the suture bridge extends beyond the width of the longitudinal ribs/runners further enhancing the strength of the suture bridge.

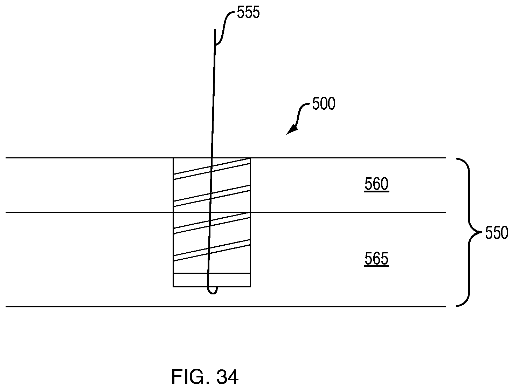

Fig. 34 shows an anchor **500** inserted into bone **550.** The anchor **500** holds one or more sutures **555** used, for example, to tie soft tissue (not shown) down to the bone **550.** The bone **550** has a hard outer layer of cortical bone **560** and soft inner layer of cancellous bone **565.** There may be more layers of bone. The number of layers, however, is not important to the following disclosure but rather there is one layer harder than the other. The hard outer layer of cortical bone or simply "cortical layer" **560** imparts a strong reactionary force on the anchor **500.** It is observed that this reactionary force is a cause of failure in anchors, particularly in anchors having an open-architecture design and made from bioabsorbable material, such as the examples described above with reference Figs. 27-33.

In testing, open-architecture anchors made from bioabsorbable material inserted into 25/5 pcf bilayer bone block simulating average humeral head bone, exhibit a novel failure mode of thread stripping from the anchors. Failure of the threads initiates in the simulated cortical layer (25 pcf) and cascades down the anchors as each subsequent thread encounters the simulated cortical layer during a pullout event, such as when a surgeon tensions a suture to tie a knot. Failure initiates in the distal most threads of the anchors because a disproportionately high amount of the (axial) load applied by the suture to the anchors is reacted by the distal most threads, which are embedded in the denser (harder) cortical layer. The failure of the distal most threads and the subsequent cascade of thread failure lead to reduced fixation strength of the anchors in average humeral head bone quality as represented by 25/5 pcf bone block.

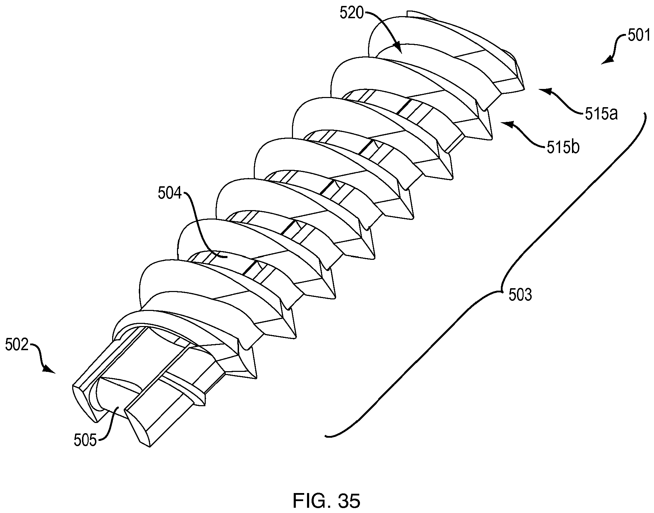

Fig. 35 shows an example of the anchor **500** designed to handle the reactionary force imparted by the cortical layer **560** and to prevent a pullout failure. The anchor **500** includes a proximal end **501** and a distal end **502.** The distal end **502** includes a suture bridge **505** that extends a partial length of the anchor **500,** such as the suture bridge **405** described above with reference to Figs. 28-31.

A majority of the anchor **500** includes screw threads **503** in the form of an open helical coil, i.e. a connected series of continuous regularly spaced turns extending in a helical or spiral form substantially from the proximal end **505a** of the suture bridge **505** to the proximal end **501** of the anchor **500.** The terms screw threads and helical coil are used interchangeably herein. The anchor **500** includes apertures **504** being defined by the space between turns of the helical coil **503.** The anchor **500** is further characterized by a number of turns per a given length, called "screw thread pitch" or simply "pitch."

At the proximal end **501** of the anchor **500,** webbing **520** extends between adjacent turns **515a** and **515b** of the coil **503.** The number of turns with webbing in between is a function of the thickness of the cortical layer **560** and the pitch of the helical coil **503.** Because the anchor **500** (and its example) is reinforced, proximally, according to the foregoing relationship, the inserted anchor **500** supports a greater axial load than compared to non-reinforced anchors. The inserted anchor **500** (and its example) exhibits a greater resistance to being pulled out of bone or "pull out strength" than compared to anchors without proximal reinforcement, particularly, in the hard layer and soft layer makeup found in typical humeral head bone stock.

In one example of the anchor **500,** the number of turns with webbing in between increases as the thickness of the cortical layer **560** and/or the pitch of helical coil 503increases.

As shown in Fig. 35, an example of the anchor **500** includes a proximal web between the distal most thread **515a** and the second most distal thread **515b.** This example of the anchor **500** has a dual lead thread meaning there are two "ridges" wrapped around the cylinder of the body of the anchor **500.** With a dual lead thread arrangement, the web **520** circumnavigates the proximal end **501** as shown. Because of the screw thread pitch of the anchor **500,** the proximal web **520** extends through the full cortical layer thickness of the humeral head bone stock providing reinforcement of the anchor through the entire cortical layer.

The example of the anchor **500** shown in Fig. 35 has one "distal" gap between the distal most thread **515a** and the second most distal thread **515b** filled with webbing **520..** Other examples of the anchor **500** may have more than one distal gap between threads filled with webbing **520.** The extent the webbing **520** progresses distally down the anchor **500** is based on the thicknesses of the cortical layer into which the anchor **500** is to be inserted and the pitch of the anchor **500.**

Some examples of the anchor **500** have different numbers of turns corresponding to different cortical layer thicknesses. The thickness of the cortical layer varies from bone to bone, e.g., the cortical layer of the humeral head is thinner than the cortical layer of the tibia. Proximal reinforcement of the anchor **500** may be advantageously tailored to a specific application e.g., the proximal reinforcement of an anchor used in shoulder repair is different than the proximal reinforcement of an anchor used in knee repair.

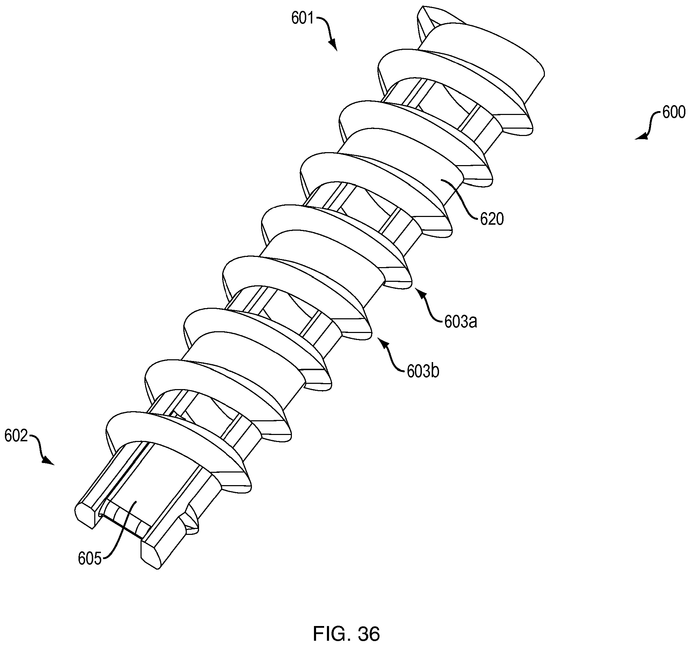

Fig. 36 shows an example anchor **600** having a proximal end 601 and distal end 602. The anchor **600** includes, at the distal end 602, a suture bridge 605 (such as one described above with reference to Figs. 27-31). The anchor **600** further includes two open helical coils **603a, 603b** in a dual lead thread arrangement. The two open helical coils **603a, 603b** extend from the suture bridge 605 toward the proximal end 601. Webbing **620** extends between adjacent turns of one of the two helical coils. The configuration of the anchor **600** strengthens/reinforces a substantial length of the anchor **600,** including the entire length. At the same time, the configuration of the anchor **600** provides a degree of openness promoting desirable bony ingrowth. It may be convenient in some examples of the anchor **600** to characterize the webbing **620** as being continuous (i.e., continuous webbing).

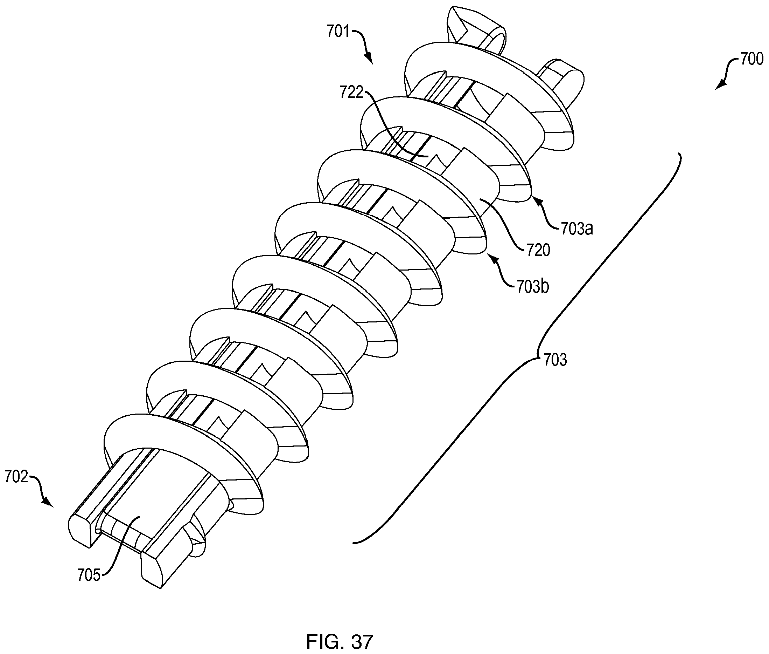

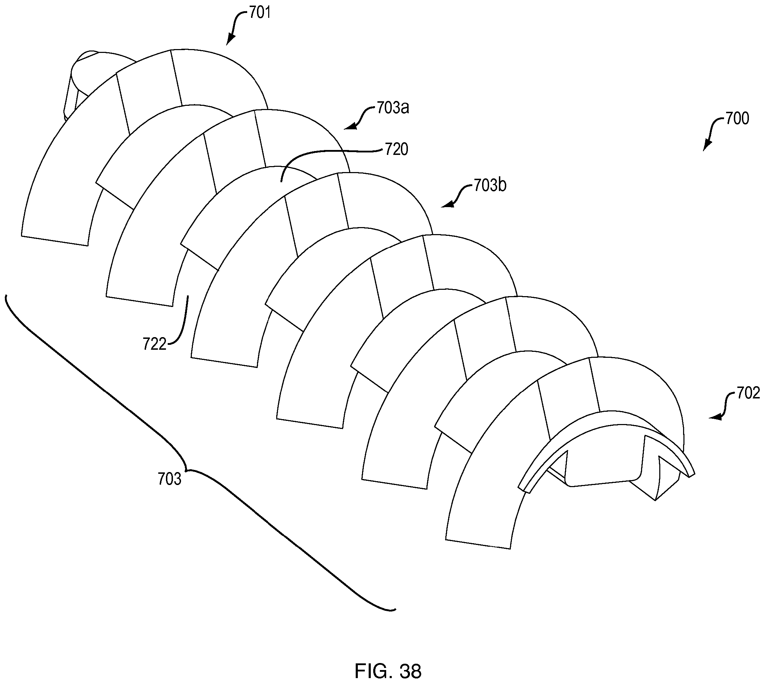

Figs. 37 and 38 show an example anchor **700** having a proximal end **701** and distal end **702.** The anchor **700** includes, at the distal end **702,** a suture bridge **705** (such as one described above with reference to Figs. 27-31). A majority of the anchor **700** includes a plurality of regularly spaced turns **703** extending in a helical or spiral form from a proximal end **705a** of the suture bridge **705,** approximately, to the proximal end **701** of the anchor **700.** Webbing **720** extends between each turn of the plurality of regularly spaced turns **703** and an adjacent turn (e.g., turns **703a** and **703b).** The webbing **720** gives the anchor **700** torsion and compression strength. The webbing **720** defines apertures **722,** which may be rectangular (square) or oval (circle) in shape. The apertures **722** give the anchor **700** a degree of openness promoting desirable bony ingrowth. It may be convenient in some examples of the anchor **700** to characterize the webbing **720** as being interrupted or perforated (i.e., interrupted/perforated webbing).

## Claims

1. A system for reconstructing a ligament comprising:
a delivery device (200) comprising a handle and shaft connected to the handle, the shaft including a distal end (201) having a slot (202) and at least two grooves (203) extending from the slot; and
an anchor (400) comprising a suture bridge (405) having a proximal end (405a) and distal end (405b) , at least two ribs (406) extending from the proximal end of the suture bridge to a proximal end of the anchor, and at least one open helical coil wrapping around the at least two ribs and extending, substantially, from the proximal end of the suture bridge to the proximal end of the anchor, the at least one open helical coil defining an internal volume communicating with a region exterior to the anchor through apertures (404) between turns of the at least one open helical coil, the anchor located on the distal end of the delivery device such that the slot houses the proximal end of the suture bridge and the at least two grooves engage the at least two ribs of the suture bridge
**characterized in that** the distal end (405b) protrudes outside the delivery device (200), the distal end (405b) of the suture bridge having a thickness greater than a thickness of the proximal end (405a), and **in that** the distal end (405b) of the suture bridge (405) is bulbous.

2. The system of any claim 1, wherein the at least one open helical coil covers the proximal end of the suture bridge.

3. The system of claim 1 or claim 2, wherein the anchor is made from a bioasborable material.

4. The system of any one of claims 1 to 3 further comprising, at the proximal end of the anchor, webbing extending between adjacent turns of the at least one helical coil, a number of turns with webbing in between being a function of the thickness of the cortical layer of bone and the pitch of the at least one helical coil.

5. The system of claim 4 wherein the number of turns with webbing in between increases with an increase in the thickness of the cortical layer of bone, increase in the pitch of the at least one helical coil or an increase in both.

6. The system of claim 4 wherein the anchor includes two open helical coils in a dual lead thread arrangement, and wherein the webbing extends between adjacent turns of one of the two helical coils.

7. The system of claim 4 wherein the at least one open helical coil includes a plurality of regularly spaced turns; and wherein the webbing extends between each pair of turns and defines openings through which the internal volume of the anchor communicates with the region exterior to the anchor.

## Patentansprüche

1. Ein System zum Rekonstruieren eines Ligaments, das Folgendes beinhaltet:
eine Liefervorrichtung (200), die einen Griff und einen Schaft, der mit dem Griff verbunden ist, beinhaltet, wobei der Schaft ein distales Ende (201) umfasst, das einen Schlitz (202) und mindestens zwei Rillen (203), die sich von dem Schlitz erstrecken,
aufweist; und
einen Anker (400), der eine Fadenbrücke (405) mit einem proximalen Ende (405a) und einem distalen Ende (405b) beinhaltet, wobei sich mindestens zwei Rippen (406) von dem proximalen Ende der Fadenbrücke zu einem proximalen Ende des Ankers erstrecken und sich mindestens eine offene schraubenförmige Spule um die mindestens zwei Rippen windet und sich im Wesentlichen von dem proximalen Ende der Fadenbrücke zu dem proximalen Ende des Ankers erstreckt, wobei die mindestens eine offene schraubenförmige Spule ein inneres Volumen definiert, das mit einem Bereich außerhalb des Ankers durch Durchlässe (404) zwischen Windungen der mindestens einen offenen schraubenförmigen Spule kommuniziert, wobei der Anker so auf dem distalen Ende der Liefervorrichtung angeordnet ist, dass der Schlitz das proximale Ende der Fadenbrücke beherbergt und die mindestens zwei Rillen in die mindestens zwei Rippen der Fadenbrücke eingreifen,
**dadurch gekennzeichnet, dass** das distale Ende (405b) außerhalb der Liefervorrichtung (200) vorsteht, wobei das distale Ende (405b) der Fadenbrücke eine Dicke aufweist, die größer als eine Dicke des proximalen Endes (405a) ist, und dass das distale Ende (405b) der Fadenbrücke (405) knollenförmig ist.

2. System gemäß Anspruch 1, wobei die mindestens eine offene schraubenförmige Spule das proximale Ende der Fadenbrücke abdeckt.

3. System gemäß Anspruch 1 oder Anspruch 2, wobei der Anker aus einem bioabsorbierbaren Material gefertigt ist.

4. System gemäß einem der Ansprüche 1 bis 3, das ferner, an dem proximalen Ende des Ankers, ein Band beinhaltet, das sich zwischen angrenzenden Windungen der mindestens einen schraubenförmigen Spule erstreckt, wobei eine Zahl von Windungen mit Band dazwischen eine Funktion der Dicke der kortikalen Knochenschicht und der Gewindesteigung der mindestens einen schraubenförmigen Spule ist.

5. System gemäß Anspruch 4, wobei die Zahl der Windungen mit Band dazwischen mit einer Zunahme der Dicke der kortikalen Knochenschicht, Zunahme der Gewindesteigung der mindestens einen schraubenförmigen Spule oder einer Zunahme von beidem zunimmt.

6. System gemäß Anspruch 4, wobei der Anker zwei offene schraubenförmige Spulen in einer doppelt geführten Gewindeanordnung umfasst, und wobei sich das Band zwischen angrenzenden Windungen von einer der zwei schraubenförmigen Spulen erstreckt.

7. System gemäß Anspruch 4, wobei die mindestens eine offene schraubenförmige Spule eine Vielzahl von regelmäßig beabstandeten Windungen umfasst; und wobei sich das Band zwischen jedem Paar Windungen erstreckt und Öffnungen definiert, durch die das innere Volumen des Ankers mit dem Bereich außerhalb des Ankers kommuniziert.

## Revendications

1. Un système pour la reconstruction d'un ligament comprenant :
un dispositif de mise en place (200) comprenant une poignée et un arbre raccordé à la poignée, l'arbre incluant une extrémité distale (201) ayant une fente (202) et au moins deux rainures (203) s'étendant à partir de la fente ; et
une ancre (400) comprenant un pont (405) de fil de suture ayant une extrémité proximale (405a) et une extrémité distale (405b), au moins deux nervures (406) s'étendant à partir de l'extrémité proximale du pont de fil de suture jusqu'à une extrémité proximale de l'ancre, et au moins un enroulement hélicoïdal ouvert enveloppant les au moins deux nervures et s'étendant, substantiellement, à partir de l'extrémité proximale du pont de fil de suture jusqu'à l'extrémité proximale de l'ancre,
l'au moins un enroulement hélicoïdal ouvert définissant un volume interne communiquant avec une région extérieure à l'ancre par des ouvertures (404) entre des spires de l'au moins un enroulement hélicoïdal ouvert, l'ancre étant située sur l'extrémité distale du dispositif de mise en place de telle sorte que la fente reçoit l'extrémité proximale du pont de fil de suture et les au moins deux rainures viennent en engagement avec les au moins deux nervures du pont de fil de suture **caractérisé en ce que** l'extrémité distale (405b) dépasse à l'extérieur du dispositif de mise en place (200), l'extrémité distale (405b) du pont de fil de suture ayant une épaisseur supérieure à une épaisseur de l'extrémité proximale (405a), et **en ce que** l'extrémité distale (405b) du pont (405) de fil de suture est renflée.

2. Le système de la revendication 1, dans lequel l'au moins un enroulement hélicoïdal ouvert recouvre l'extrémité proximale du pont de fil de suture.

3. Le système de la revendication 1 ou de la revendication 2, dans lequel l'ancre est réalisée à partir d'un matériau bioabsorbable.

4. Le système de n'importe laquelle des revendications 1 à 3 comprenant en outre, au niveau de l'extrémité proximale de l'ancre, une bande s'étendant entre des spires adjacentes de l'au moins un enroulement hélicoïdal, un nombre de spires avec une bande entre elles étant fonction de l'épaisseur de la couche corticale d'os et du pas de l'au moins un enroulement hélicoïdal.

5. Le système de la revendication 4 dans lequel le nombre de spires avec une bande entre elles augmente avec une augmentation de l'épaisseur de la couche corticale d'os, une augmentation du pas de l'au moins un enroulement hélicoïdal ou une augmentation des deux.

6. Le système de la revendication 4 dans lequel l'ancre inclut deux enroulements hélicoïdaux ouverts selon un agencement de filetage à double filet, et dans lequel la bande s'étend entre des spires adjacentes d'un des deux enroulements hélicoïdaux.

7. Le système de la revendication 4 dans lequel l'au moins un enroulement hélicoïdal ouvert inclut une pluralité de spires espacées de manière régulière ; et dans lequel la bande s'étend entre chaque paire de spires et définit des espaces libres par lesquels le volume interne de l'ancre communique avec la région extérieure à l'ancre.
